(19) Europäisches Patentamt
European Patent Office
Office européen des brevets

(11) Numéro de publication: **0 571 511 B1**

# (12) FASCICULE DE BREVET EUROPEEN

(45) Date de publication de fascicule du brevet: **07.12.94**

(51) Int. Cl.$^5$: **C07D 211/26**, C07D 405/12, A61K 31/445

(21) Numéro de dépôt: **92906408.7**

(22) Date de dépôt: **12.02.92**

(86) Numéro de dépôt internationale : **PCT/FR92/00131**

(87) Numéro de publication internationale : **WO 92/14710 (03.09.92 92/23)**

## (54) NOUVELLES UREES ET THIOUREES, LEUR PREPARATION ET LEUR APPLICATION EN THERAPEUTIOUE.

(30) Priorité: **14.02.91 FR 9101740**

(43) Date de publication de la demande: **01.12.93 Bulletin 93/48**

(45) Mention de la délivrance du brevet: **07.12.94 Bulletin 94/49**

(84) Etats contractants désignés: **AT BE CH DE DK ES FR GB GR IT LI LU MC NL SE**

(56) Documents cités:
**EP-A- 0 199 400**
**EP-A- 0 229 391**
**US-A- 4 071 524**

**Chemical Abstracts, vol. 114, 1991, page 757, abrégé no. 143155n, Columbus, Ohio, US; Orjales Venero A.: "Preparation of new antihistaminic 4-piperidinealkyl urea derivatives"**

**Journal of Medicinal Chemistry, vol. 33, no. 7, 1990, pages 1880-1887, Washington, US; Sugitomo H. et al.: "Novel piperidine derivatives. Synthesis and anti-acetylchloinesterase activity of 1-benzyl-4-[-2-(N-benzoylamino)ethyl]piperidine derivatives"**

(73) Titulaire: **PIERRE FABRE MEDICAMENT**
**45, Place Abel Gance**
**F-92100 Boulogne (FR)**

(72) Inventeur: **VIDALUC, Jean-Louis**
**Jean-Petit**
**F-81100 Castres (FR)**
Inventeur: **BIGG, Denis**
**122, avenue de Lavaur**
**F-81100 Castres (FR)**

(74) Mandataire: **Martin, Jean-Jacques**
**Cabinet REGIMBEAU**
**26, Avenue Kléber**
**F-75116 Paris (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

EP 0 571 511 B1

## Description

La présente invention, réalisée au Centre de Recherche Pierre Fabre, a pour objet de nouvelles urées et thiourées, leur préparation et leur application en thérapeutique.

L'état de la technique antérieure peut être illustré par ES-A-2 007 808 qui vise un procédé de préparation de dérivés de 4-pipéridine-alkylurées dont les structures et activités sont cependant différentes de celles de la précédente demande. Cet état de la technique peut en outre être complété par J. Med. Chem. 1990, 33, 1880 et EP-A-229 391 qui enseignent des composés pharmacologiquement approchant de ceux de la présente invention, mais qui présentent par rapport à eux des différences structurales fondamentales.

Les composés de l'invention répondent à la formule générale 1 :

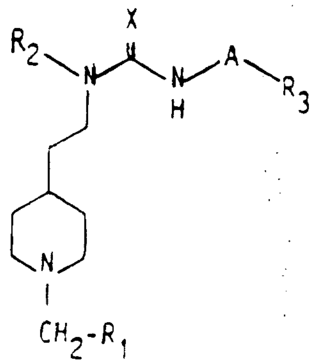

1

dans laquelle :

- $R_1$ représente un groupe cycloalkyle en $C_5$-$C_7$, un groupe phényle ou un radical phényle substitué par un groupe alkyle en $C_1$-$C_4$, un groupe alkoxy en $C_1$-$C_4$, ou un atome d'halogène :
- $R_2$ représente un atome d'hydrogène ou un groupe alkyle en $C_1$-$C_4$ ;
- X représente un atome d'oxygène ou un atome de soufre .
- A représente un radical méthylène, un radical carbonyle, ou un radical sulfonyle ;
- $R_3$ représente :
  - un groupe aryle de formule :

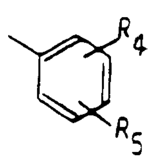

où $R_4$ et $R_5$, indépendamment l'un de l'autre, représentent un atome d'hydrogène ou d'halogène, un groupe nitro, un groupe alkyle en $C_1$-$C_4$, un groupe alkoxy en $C_1$-$C_4$, un groupe acyle en $C_1$-$C_4$, un groupe benzoyle, un groupe alkylsulfonyle en $C_1$-$C_4$, ou un groupe trifluorométhyle ;

ou

$R_4$ et $R_5$ pris ensemble représentent un groupement méthylènedioxy ;

  - un groupe oxofluorényle de formule :

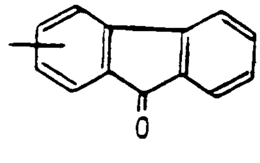

- un groupe dioxoanthracényle de formule :

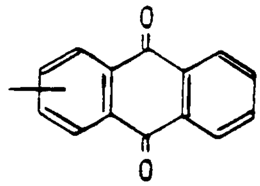

à la condition toutefois que A ne représente pas le radical méthylène lorsque X représente un atome d'oxygène.

L'invention couvre également les sels des composés de formule générale 1 avec des acides pharmaceutiquement acceptables.

Les composés de formule générale 1 de l'invention peuvent être préparés selon le schéma de réaction suivant :

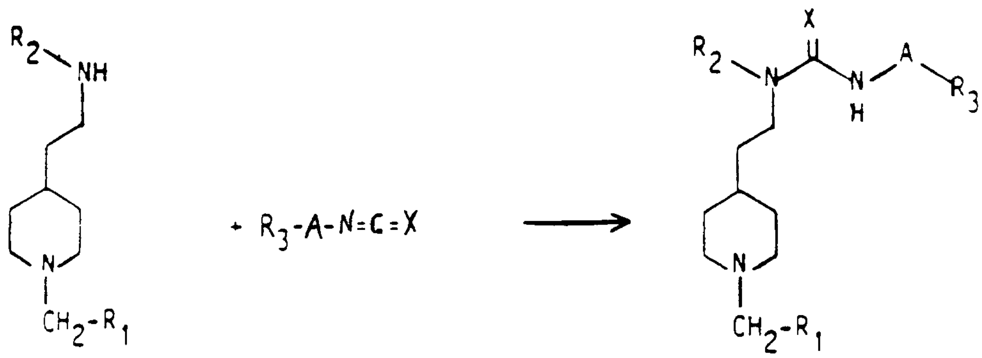

2 3 1

où $R_1$, $R_2$, $R_3$, A et X sont définis comme ci-dessus.

Les amines de départ 2 et les hétérocumulènes de formule 3 peuvent être obtenus selon des méthodes connues. La réaction des composés 2 avec les composés 3 peut être effectuée à la température ambiante ou accélérée par simple chauffage. La réaction s'effectue, de préférence, en solution. Le solvant employé peut être, à titre d'exemple, un solvant chloré tel que le dichlorométhane ou le dichloroéthane, un solvant cétonique tel que l'acétone, ou un éther tel que la tétrahydrofurane. Lorsque le composé de formule 3 est peu réactif, un solvant alcoolique peut également être employé.

Les exemples suivants illustrent l'invention sans toutefois en limiter la portée.
Les analyses et les spectres IR et RMN confirment la structure des composés obtenus selon l'invention.

Exemple 1

Benzoyl-1 méthyl-3 [(benzyl-1 pipéridinyl-4) éthyl-2]-3 thiourée : composé n°1 ; $R_1 = C_6H_5$, $R_2$ = Me, X = S, A = CO, $R_3 = C_6H_5$.

On ajoute 0,5 ml d'isothiocyanate de benzoyle à une solution de 0,8 g de (méthylamino-2 éthyl)-4 benzyl-1 pipéridine dans 10 ml de dichlorométhane. On agite à la température ambiante pendant une heure. On évapore à sec et on reprend le produit dans de l'acétate d'éthyle. On extrait avec une solution aqueuse d'acide chlorhydrique 2N. La phase aqueuse est alcalinisée et extraite par le chloroforme. Après lavage à la saumure et séchage sur sulfate de sodium, le solvant est évaporé pour fournir le composé 1 sous forme d'huile jaune pâle. On traite l'huile avec une solution alcoolique d'acide fumarique et l'on obtient le fumarate du composé 1 sous forme de cristaux blancs (0,45 g).
PF° : 163-5°C.

Exemple 2

Benzyl-1 [(benzyl-1 pipéridinyl-4) éthyl-2]-3 thiourée : composé n° 2 ; $R_1 = C_6H_5$, $R_2 = H$, X = S, A = $CH_2$, $R_3 = C_6H_5$.

Une solution de 2,2 g de (amino-2 éthyle)-4 benzyl-1 pipéridine et 1,4 ml d'isothiocyanate de benzyle dans 20 ml d'éthanol absolu est chauffée à reflux pendant une heure. Le solvant est évaporé et l'huile orangée obtenue purifiée sur colonne de silice éluée par un mélange chloroforme/méthanol (95/5). L'huile obtenue est traitée par une solution alcoolique d'acide chlorhydrique et le chlorhydrate du composé 2 est précipité par l'addition d'éther éthylique. On obtient 3.4 g de cristaux blancs.
PF° : 198-200° C.

Exemple 3

Tosyl-1 [(benzyl-1 pipéridinyl-4) éthyl-2]-3 urée : composé n° 3 ; $R_1 = C_6H_5$, $R_2 = H$, X = O, A = $SO_2$, $R_3$ = 4-Me.$C_6H_4$

On ajoute 0,73 ml d'isocyanate de (méthyl-4 phényl) sulfonyle à une solution de 1 g de (amino-2 éthyl)-4 benzyl-1 pipéridine dans 10 ml de dichlorométhane avec refroidissement (bain de glace). Après une heure, on laisse le mélange réactionnel revenir à la température ambiance. On filtre le solide blanc que l'on lave au dichlorométhane. Après séchage sous vide à 50° C on obtient 1,4 g du composé 3 sous forme de cristaux blancs.
PF° : 155-6° C.

Le tableau 1 ci-après résume les principaux produits synthétisés qui illustrent l'invention sans toutefois en limiter la portée.

**Tableau 1**

| Comp n° | $R_1$ | $R_2$ | X | A | $R_3$ | Sel | PF(°C) |
|---|---|---|---|---|---|---|---|
| 1 | $C_6H_5$ | Me | S | CO | $C_6H_5$ | fumarate | 163-5 |
| 2 | $C_6H_5$ | H | S | $CH_2$ | $C_6H_5$ | HCl | 198-200 |
| 3 | $C_6H_5$ | H | O | $SO_2$ | $4-Me.C_6H_4$ | base libre | 155-6 |
| 4 | $C_6H_5$ | H | S | CO | $C_6H_5$ | HCl | 182-4 |
| 5 | $C_6H_5$ | H | S | CO | $3-NO_2.C_6H_4$ | HCl | 162-4 |
| 6 | $C_6H_5$ | H | S | CO | $4-MeCO.C_6H_4$ | fumarate | 202-4 |
| 7 | $C_6H_5$ | H | S | CO | $2-NO_2.C_6H_4$ | HCl | 166-8 |
| 8 | $C_6H_5$ | H | S | CO | $4-C_6H_5CO.C_6H_4$ | HCl | 206-8 |
| 9 | $C_6H_5$ | H | S | CO | 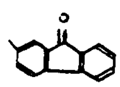 | HCl | 247-50 |
| 10 | $C_6H_5$ | H | S | CO | $4-CF_3.C_6H_4$ | fumarate | 204-5 |
| 11 | $C_6H_5$ | H | S | CO | $4-MeSO_2.C_6H_4$ | fumarate | 193-5 |
| 12 | $C_6H_5$ | H | S | CO | $3,4-(OCH_2O).C_6H_3$ | HCl | 176-8 |
| 13 | $C_6H_5$ | H | S | CO | 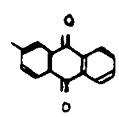 | HCl | 238-40 |
| 14 | $C_6H_5$ | Et | S | CO | $C_6H_5$ | HCl | 143-5 |
| 15 | $C_6H_5$ | Et | S | $CH_2$ | $C_6H_5$ | HCl | 135-40 |
| 16 | $C_6H_5$ | H | S | CO | $4-MeO.C_6H_4$ | HCl | 215-20 |

**Tableau 1 (suite)**

| Comp n° | $R_1$ | $R_2$ | X | A | $R_3$ | Sel | PF(°C) |
|---|---|---|---|---|---|---|---|
| 17 | $C_6H_5$ | H | S | CO | $4-Cl.C_6H_4$ | HCl | 195-200 |
| 18 | $C_6H_5$ | H | O | CO | $C_6H_5$ | HCl | 220-3 |
| 19 | $cC_6H_{11}$ | H | S | CO | $C_6H_5$ | base libre | 144-6 |

Les composés de l'invention ont été soumis à des essais pharmacologiques qui ont montré leur intérêt en tant qu'inhibiteurs de l'acétylcholinestérase.

A cet effet, les composés ont été étudiés selon la méthode décrite par G.L. Ellman et al., Biochem. Pharmacol. 7, 88-95 (1961).

Les résultats obtenus sur certains composés de l'invention sont reportés, à titre d'exemple, dans le tableau 2.

Tableau 2

| Inhibition de l'activité de l'acétylcholinestérase | |
|---|---|
| Composé n° | $IC_{50}$ (nM) |
| 5 | 1,5 |
| 6 | 7,5 |
| 7 | 40 |
| 8 | 14 |
| 10 | 33 |
| 12 | 18 |
| 15 | 260 |
| Tacrine | 120 |

Les composés de l'invention sont des inhibiteurs de l'acétylcholinestérase ; aussi, ils peuvent être utiles dans le traitement des maladies telles que la myasthénie, les troubles de la mémoire, les démences, telles que les démences séniles ou la maladie d'Alzheimer.

Les préparations pharmaceutiques contenant ces principes actifs peuvent être mises en forme pour l'administration par voie orale, rectale, parentérale ou locale, par exemple sous la forme de capsules, comprimés, granulés, gélules, solutés liquides, sirops ou suspensions buvables, et contenir les excipients appropriés.

Il est également possible d'y associer d'autres principes actifs pharmaceutiquement et thérapeutiquement acceptables.

**Revendications**

1. Urées et thiourées correspondant à la formule générale 1:

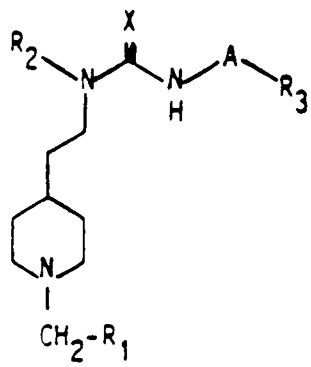

1

dans laquelle :

$R_1$ représente un groupe cycloalkyle en $C_5$-$C_7$, un groupe phényle ou un radical phényle substitué par un groupe alkyle en $C_1$-$C_4$, un groupe alkoxy en $C_1$-$C_4$, ou un atome d'halogène ;

$R_2$ représente un atome d'hydrogène ou a groupe alkyle en $C_1$-$C_4$ ;

X représente un atome d'oxygène ou un atome de soufre ;

A représente un radical méthylène, un radical carbonyle, ou un radical sulfonyle ;

$R_3$ représente :

- un groupe aryle de formule :

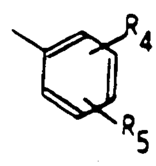

où $R_4$ et $R_5$, indépendamment l'un de l'autre, représentent un atome d'hydrogène ou d'halogène, un groupe nitro, un groupe alkyle en $C_1$-$C_4$, un groupe alkoxy en $C_1$-$C_4$, un groupe acyle en $C_1$-$C_4$, un groupe benzoyle, un groupe alkylsulfonyle en $C_1$-$C_4$, ou un groupe trifluorométhyle ;

ou

$R_4$ et $R_5$ pris ensemble représentent un groupement méthylènedioxy ;

- un groupe oxofluorényle de formule :

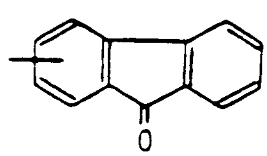

- un groupe dioxoanthracényle de formule :

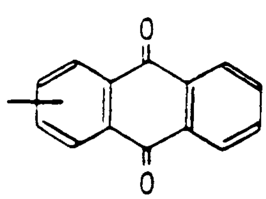

à la condition toutefois que A ne représente pas le radical méthylène lorsque X représente un atome d'oxygène.

ainsi que les sels organiques ou minéraux thérapeutiquement acceptables de ces molécules.

**2.** Composés de formule générale 1 selon la revendication 1 caractérisés en ce que X représente un atome de soufre.

**3.** Composés de formule générale 1 selon les revendications 1 et 2, caractérisés en ce que A représente un groupe carbonyle.

**4.** Composés de formule générale 1 selon la revendication 1 caractérisés par le fait qu'ils sont choisis parmi :
- Benzoyl-1 méthyl-3 [(benzyl-1 pipéridinyl-4) éthyl-2]-3 thiourée
- Benzyl-1 [(benzyl-1 pipéridinyl-4) éthyl-2]-3 thiourée
- Tosyl-1 [(benzyl-1 pipéridinyl-4) éthyl-2]-3 urée
- Benzoyl-1 [(benzyl-1 pipéridinyl-4) éthyl-2]-3 thiourée
- (Nitro-3 benzoyl)-1 [(benzyl-1 pipéridinyl-4) éthyl-2]-3 thiourée
- (Méthoxy-4 benzoyl)-1 [(benzyl-1 pipéridinyl-4) éthyl-2]-3 thiourée
- (Nitro-2 benzoyl) -1 [(benzyl-1 pipéridinyl-4) ethyl-2]-3 thiourée
- (Benzoyl-4 benzoyl)-1 [(benzyl-1 pipéridinyl-4) éthyl-2]-3 thiourée
- (Oxo-9 fluorènoyl-2)-1 [(benzyl-1 pipéridinyl-4) éthyl-2]-3 thiourée
- (Trifluorométhyl-4 benzoyl)-1 [(benzyl-1 pipéridinyl-4) éthyl-2]-3 thiourée
- (Méthylsulfonyl-4 benzoyl)-1 [(benzyl-1 pipéridinyl-4) éthyl-2]-3 thiourée
- (Méthylènedioxy-3,4 benzoyl)-1 [(benzyl-1 pipéridinyl-4) éthyl-2]-3 thiourée
- (Dioxo-9,10 anthracénoyl-2)-1 [(benzyl-1 pipéridinyl-4) éthyl-2]-3 thiourée
- Benzoyl-1 éthyl-3 [(benzyl-1 pipéridinyl-4) éthyl-2]-3 thiourée
- Benzyl-1 éthyl-3 [(benzyl-1 pipéridinyl-4) èthyl-2]-3 thiourée
- (Méthoxy-4 benzoyl)-1 [(benzyl-1 piperidinyl-4) éthyl-2]-3 thiourée
- (Chloro-4 benzoyl)-1 [(benzyl-1 pipéridinyl-4) éthyl-2]-3 thiourée
- Benzoyl-1 [(benzyl-1 pipéridinyl-4) éthyl-2]-3 urée
- Benzoyl-1 [(cyclohexylméthyl-1 pipéridinyl-4) éthyl-2]-3 thiourée

**5.** Procédé de préparation de composés selon les revendications 1 à 4 caractérisé en ce que l'on fait réagir une amine de formule générale 2 avec un composé de formule générale 3 :

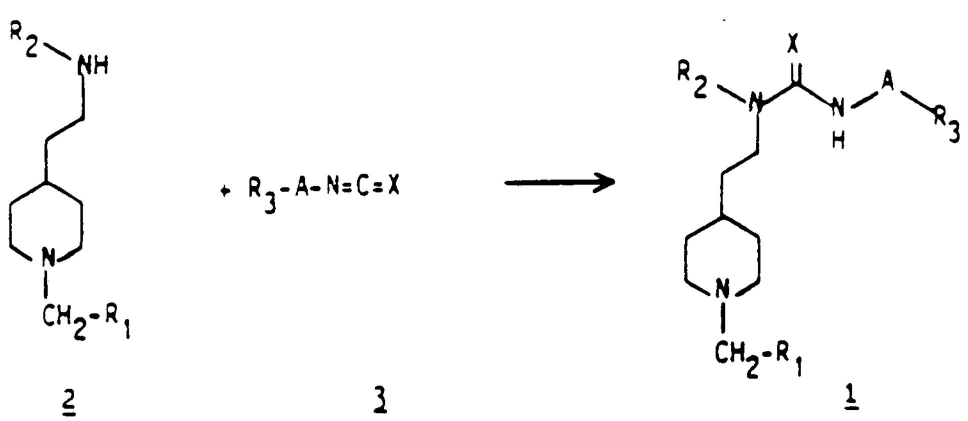

2 3 1

où $R_1$, $R_2$, $R_3$, A, X, sont définis comme ci-dessus.

**6.** Procédé de préparation de composés chimiques selon la revendication 5 caractérisé en ce que la réaction des composés de formule générale 2 avec les composés de formule générale 3 s'effectue en solution. Le solvant employé peut être, à titre d'exemple, un solvant chloré tel que le dichlorométhane ou le dichloroéthane, un solvant cétonique tel que l'acétone, ou un éther tel que la tétrahydrofurane.

**7.** A titre de médicaments nouveaux utiles, par exemple, dans le traitement de la myasthénie, des troubles de la mémoire, des démences, telles que les démences séniles ou la maladie d'Alzheimer, les composés définis selon l'une des revendications 1 à 4.

**8.** Composition pharmaceutique caractérisée en ce qu'elle contient un composé de l'une des revendications 1 à 4.

**9.** Composition pharmaceutique caractérisée en ce qu'elle contient un composé de l'une des revendications 1 à 4 en association avec tout excipient approprié.

**10.** Composition pharmaceutique caractérisée en ce qu'elle contient un composé de l'une des revendications 1 à 4 associé à un autre principe actif.

## Claims

**1.** Ureas and thioureas corresponding to the general formula 1:

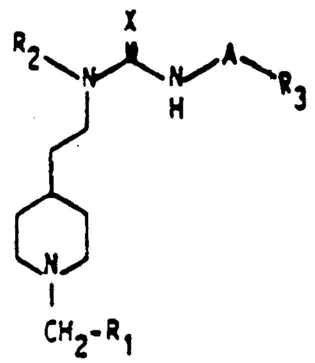

1.

in which:

- $R_1$ represents a $C_5$-$C_7$ cycloalkyl group, a phenyl group or a phenyl radical substituted by a $C_1$-$C_4$ alkyl group, a $C_1$-$C_4$ alkoxy group or a halogen atom;
- $R_2$ represents a hydrogen atom or a $C_1$-$C_4$ alkyl group;
- X represents an oxygen atom or a sulfur atom;
- A represents a methylene radical, a carbonyl radical or a sulfonyl radical;
- $R_3$ represents:
  - an aryl group of formula:

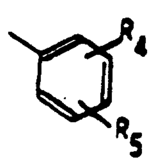

where $R_4$ and $R_5$, independently of each other, represent a hydrogen or halogen atom, a nitro group, a $C_1$-$C_4$ alkyl group, a $C_1$-$C_4$ alkoxy group, a $C_1$-$C_4$ acyl group, a benzoyl group, a $C_1$-$C_4$ alkylsulfonyl group, or a trifluoromethyl group;
or
$R_4$ and $R_5$ taken together represent a methylenedioxy group;

- an oxofluorenyl group of formula:

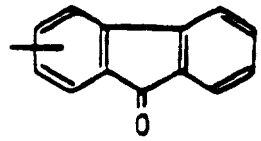

- a dioxoanthracenyl group of formula:

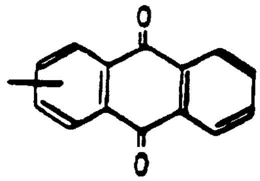

on the condition however that A does not represent a methylene radical when X represents an oxygen atom,

as well as the therapeutically acceptable organic or inorganic salts of these molecules.

**2.** Compounds of general formula 1 according to Claim 1, characterized in that X represents a sulfur atom.

**3.** Compounds of general formula 1 according to Claims 1 and 2, characterized in that A represents a carbonyl group.

**4.** Compounds of general formula 1 according to Claim 1, characterized by the fact that they are chosen from:

- 1-benzoyl-3-methyl-3-[2-(1-benzyl-4-piperidyl)ethyl]thiourea
- 1-benzyl-3-[2-(1-benzyl-4-piperidyl)ethyl]thiourea
- 1-tosyl-3-[2-(1-benzyl-4-piperidyl)ethyl]urea
- 1-benzoyl-3-[2-(1-benzyl-4-piperidyl)ethyl]thiourea
- 1-(3-nitrobenzoyl)-3-[2-(1-benzyl-4-piperidyl)ethyl]thiourea
- 1-(4-methoxybenzoyl)-3-[2-(1-benzyl-4-piperidyl)ethyl]thiourea
- 1-(2-nitrobenzoyl)-3-[2-(1-benzyl-4-piperidyl)ethyl]thiourea
- 1-(4-benzoylbenzoyl)-3-[2-(1-benzyl-4-piperidyl)ethyl]thiourea
- 1-(9-oxo-2-fluorenoyl)-3-[2-(1-benzyl-4-piperidyl)ethyl]thiourea
- 1-(4-trifluoromethylbenzoyl)-3-[2-(1-benzyl-4-piperidyl)ethyl]thiourea
- 1-(4-methylsulfonylbenzoyl)-3-[2-(1-benzyl-4-piperidyl)ethyl]thiourea
- 1-(3,4-methylenedioxybenzoyl)-3-[2-(1-benzyl-4-piperidyl)ethyl]thiourea
- 1-(9,10-dioxo-2-anthracenoyl)-3-[2-(1-benzyl-4-piperidyl)ethyl]thiourea
- 1-benzoyl-3-ethyl-3-[2-(1-benzyl-4-piperidyl)ethyl]thiourea
- 1-benzyl-3-ethyl-3-[2-(1-benzyl-4-piperidyl)ethyl]thiourea
- 1-(4-methoxybenzoyl)-3-[2-(1-benzyl-4-piperidyl)ethyl]thiourea
- 1-(4-chlorobenzoyl)-3-[2-(1-benzyl-4-piperidyl)ethyl]thiourea
- 1-benzoyl-3-[2-(1-benzyl-4-piperidyl)ethyl]urea
- 1-benzoyl-3-[2-(1-cyclohexylmethyl-4-piperidyl)ethyl]thiourea.

**5.** Process for preparing the compounds according to Claims 1 to 4, characterized in that an amine of general formula 2 is reacted with a compound of general formula 3:

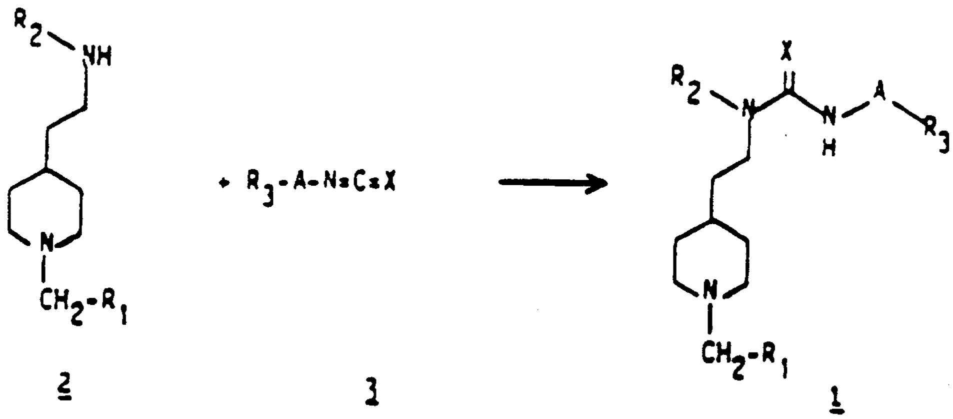

2 3 1

where $R_1$, $R_2$, $R_3$, A, X are defined as above.

**6.** Process for preparing chemical compounds according to Claim 5 characterized in that the reaction of the compounds of general formula 2 with the compounds of general formula 3 is performed in solution. The solvent used may be, by way of example, a chlorinated solvent such as dichloromethane or dichloroethane, a ketonic solvent such as acetone or an ether such as tetrahydrofuran.

**7.** The compounds defined according to one of Claims 1 to 4 as novel medicinal products useful for example in the treatment of myasthenia, memory disorders, dementias such as senile dementias or Alzheimer's disease.

**8.** Pharmaceutical composition characterized in that it contains a compound of one of Claims 1 to 4.

**9.** Pharmaceutical composition characterized in that it contains a compound of one of Claims 1 to 4 in combination with any appropriate excipient.

**10.** Pharmaceutical composition characterized in that it contains a compound of one of Claims 1 to 4 combined with another active ingredient.

## Patentansprüche

**1.** Harnstoffe und Thioharnstoffe der allgemeinen Formel (1)

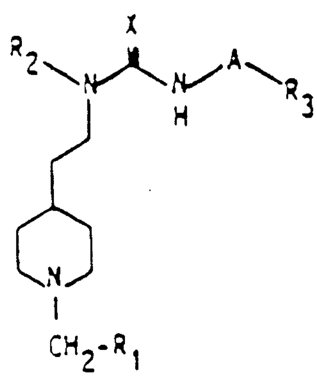

1

worin bedeuten:

- $R_1$ eine $C_5$-$C_7$-Cycloalkylgruppe, eine Phenylgruppe oder einen Phenylrest, der substituiert ist durch eine $C_1$-$C_4$-Alkylgruppe, eine $C_1$-$C_4$-Alkoxygruppe oder ein Halogenatom;
- $R_2$ ein Wasserstoffatom oder eine $C_1$-$C_4$-Alkylgruppe;
- X ein Sauerstoffatom oder ein Schwefelatom;

A einen Methylenrest, einen Carbonylrest oder einen Sulfonylrest;
$R_3$
- eine Arylgruppe der Formel

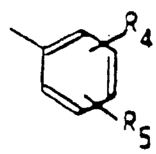

worin $R_4$ und $R_5$ unabhängig voneinander ein Wasserstoffatom oder ein Halogenatom, eine Nitrogruppe, eine $C_1$-$C_4$-Alkylgruppe, eine $C_1$-$C_4$-Alkoxygruppe, eine $C_1$-$C_4$-Acylgruppe, eine Benzoylgruppe, eine $C_1$-$C_4$-Alkylsulfonylgruppe oder eine Trifluoromethylgruppe darstellen; oder worin $R_4$ und $R_5$ zusammengenommen eine Methylendioxygruppe darstellen;
- eine Oxofluorenyl-Gruppe der Formel

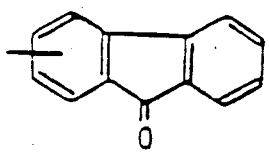

- eine Dioxoanthracenylgruppe der Formel

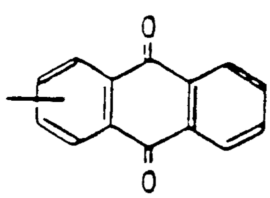

jedoch mit der Maßgabe, daß A keinen Methylenrest darstellt, wenn X für ein Sauerstoffatom steht, sowie die therapeutisch akzeptablen organischen oder anorganischen (mineralischen) Salze dieser Moleküle.

**2.** Verbindungen der allgemeinen Formel (1) nach Anspruch 1, dadurch gekennzeichnet, daß X für ein Schwefelatom steht.

**3.** Verbindungen der allgemeinen Formel (1) nach den Ansprüchen 1 und 2, dadurch gekennzeichnet, daß A für eine Carbonylgruppe steht.

**4.** Verbindungen der allgemeinen Formel (1) nach Anspruch 1, dadurch gekennzeichnet, daß sie ausgewählt werden aus der Gruppe:
- 1-Benzoyl-3-methyl-3-[(1-benzyl-4-piperidinyl)-2-ethyl]thioharnstoff
- 1-Benzyl-3-[(1-benzyl-4-piperidinyl)-2-ethyl]thioharnstoff
- 1-Tosyl-3-[(1-benzyl-4-piperidinyl)-2-ethyl]harnstoff
- 1-Benzoyl-3-[(1-benzyl-4-piperidinyl)-2-ethyl]thioharnstoff
- 1-(3-Nitrobenzoyl)-3-[(1-benzyl-4-piperidinyl)-2-ethyl]thioharnstoff
- 1-(4-Methoxy-benzoyl)-3-[(1-benzyl-4-piperidinyl)-2-ethyl]thioharnstoff
- 1-(2-Nitro-benzoyl)-3-[(1-benzyl-4-piperidinyl)-2-ethyl]thioharnstoff
- 1-(4-Benzoyl-benzoyl)-3-[(1-benzyl-4-piperidinyl)-2-ethyl]thioharnstoff
- 1-(9-Oxo-2-fluorenoyl)-3-[(1-benzyl-4-piperidinyl)-2-ethyl]thioharnstoff
- 1-(4-Trifluoromethyl-benzoyl)-3-[(1-benzyl-4-piperidinyl)-2-ethyl]thioharnstoff
- 1-(4-Methylsulfonyl-benzoyl)-3-[(1-benzyl-4-piperidinyl)-2-ethyl]thioharnstoff
- 1-(3,4-Methylendioxy-benzoyl)-3-[1-benzyl-4-piperidinyl)-2-ethyl]thioharnstoff
- 1-(9,10-Dioxo-2-anthracenoyl)-3-[(1-benzyl-4-piperidinyl)-2-ethyl]thioharnstoff

- 1-Benzoyl-3-ethyl-3-[(1-benzyl-4-piperidinyl)-2-ethyl]thioharnstoff
- 1-Benzyl-3-ethyl-3-[(1-benzyl-4-piperidinyl)-2-ethyl]thioharnstoff
- 1-(4-Methoxy-benzoyl)-3-[(1-benzyl-4-piperidinyl)-2-ethyl]thioharnstoff
- 1-(4-Chloro-benzoyl)-3-[(1-benzyl-4-piperidinyl)-2-ethyl]thioharnstoff
- 1-Benzoyl-3-[(1-benzyl-4-piperidinyl)-2-ethyl]harnstoff
- 1-Benzoyl-3-[(1-cyclohexylmethyl-4-piperidinyl)-2-ethyl]thioharnstoff.

**5.** Verfahren zur Herstellung der Verbindungen nach den Ansprüchen 1 bis 4, dadurch gekennzeichnet, daß man ein Amin der allgemeinen Formel (2) mit einer Verbindung der allgemeinen Formel (3) reagieren läßt

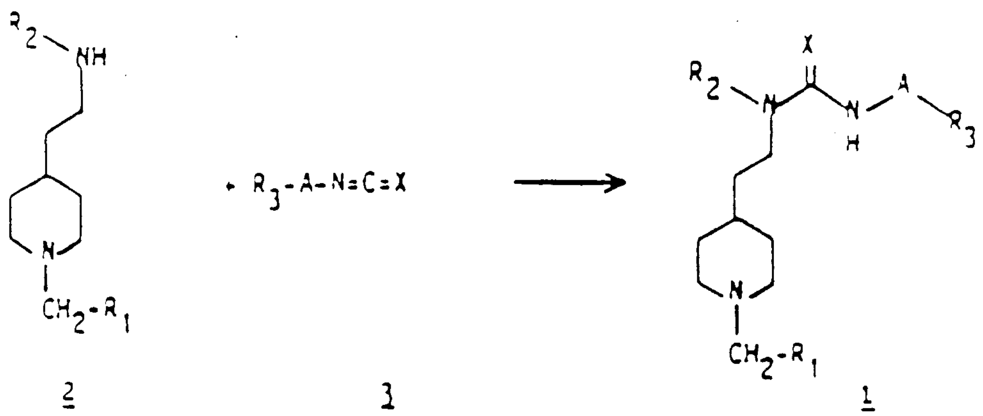

2 3 1

worin $R_1$, $R_2$, $R_3$, A und X wie oben definiert sind.

**6.** Verfahren zur Herstellung der chemischen Verbindungen nach Anspruch 5, dadurch gekennzeichnet, daß die Umsetzung der Verbindungen der allgemeinen Formel (2) mit den Verbindungen der allgemeinen Formel (3) in Lösung durchgeführt wird, wobei das verwendete Lösungsmittel beispielsweise ein chloriertes Lösungsmittel wie Dichlormethan oder Dichlorethan, ein Ketonlösungsmittel wie Aceton oder ein Äther wie Tetrahydrofuran sein kann.

**7.** Verbindungen nach einem der Ansprüche 1 bis 4 als neue Arzneimittel, die beispielsweise verwendbar (nützlich) sind für die Behandlung der Myasthenie, von Gedächtnisstörungen, Dementien, wie Altersdementien oder der Alzheimer-Krankheit.

**8.** Pharmazeutische Zusammensetzung, dadurch gekennzeichnet, daß sie eine Verbindung nach einem der Ansprüche 1 bis 4 enthält.

**9.** Pharmazeutische Zusammensetzung, dadurch gekennzeichnet, daß sie eine Verbindung nach einem der Ansprüche 1 bis 4 in Kombination mit irgendeinem geeigneten Exzipienten enthält.

**10.** Pharmazeutische Zusammensetzung, dadurch gekennzeichnet, daß sie eine Verbindung nach einem der Ansprüche 1 bis 4 in Assoziation mit einem anderen Wirkstoff enthält.